# EUROPEAN PATENT APPLICATION

(11) **EP 4 155 000 A1**
(43) Date of publication of application: **29.03.2023**
(21) Application number: 22819888.3
(22) Date of filing: 23.03.2022
(51) Int. Cl.: B09B 3/70, B09B 5/00

(54) **BIODEGRADABLE PLASTIC PRODUCT PRODUCTION METHOD AND PRODUCTION SYSTEM**

(30) Priority: 09.06.2021 JP 2021096844
(71) Applicant: Nishi Nihon Ecology Corp., Kumamoto-shi, Kumamoto 861-8037 (JP)
(72) Inventor: AOKI, Toshiaki, Kumamoto-shi, Kumamoto 861-8038 (JP); AOKI, Hideaki, Kumamoto-shi, Kumamoto 861-8039 (JP); AOKI, Takanori, Kumamoto-shi, Kumamoto 861-8002 (JP); AOKI, Hironori, Oita-shi, Oita 870-1183 (JP)
(74) Representative: Herrero & Asociados, S.L.
(86) International application number: PCT/JP2022/013736
(87) International publication number: WO 2022/259698

(57) **Abstract**

The present invention is to provide a method and a system of manufacturing a biodegradable plastic product that is made of an organic substance as raw material and biodegraded in a general soil environment for a short period. The method and the system according to the present invention crushes organic waste material, adds an inorganic treatment agent and a dehydrating treatment agent to the crushed organic waste material, stirs the mixture to dehydrate and solidify the organic waste material, dries the solidified organic waste material and powders the dried organic waste material to microparticles, subjects the powdered organic waste material to poly-lactic acid generation, and molds a biodegradable plastic product with molding equipment by using the poly-lactic acid organic waste material as raw material.

## Description

### TECHNICAL FIELD

The present invention relates to a method and a system of manufacturing a biodegradable plastic product composed of an organic substance.

### BACKGROUND

Conventional plastics products are derived from petroleum, which are disposed through incineration, landfill, etc. because they cannot decompose into soil and water. Some of them are illegally disposed into the sea to cause environmental damage.

However, the world society is called to decarbonize the economy. Even in our country, plastics products are being switched to bioplastic or green plastic products as a part of realization of a carbon-neutral society.

For example, the bioplastic products containing a petroleum-based content of 75% or more are known. However, such biodegradable plastic products are known to be degradable in only a special environment in hot and humid conditions, which are partially degraded even in a special general indoor or soil environment for long periods of at least five years (Non-Patent Document 1).

On the other hand, organic waste material discharged from manufacturing industries, for example, shochu (distilled spirit) lees are subjected to methane fermentation or processed into feed (Non-Patent Document 2).

### Document in the existing art

Non-Patent Document 1: NATIONAL GEOGRAPHIC, "Is it true that bioplastics are environment-friendly?", November 20, 2018. Retrieval on May 29, 2021, Internet < https://natgeo.nikkeibp.co.jp/atcl/news/18/111900500/>
Non-Patent Document 2: Food and Container Vol.57, November, 2016, "Utilization of lees (e.g. shochu lees) generated in shochu manufacturing process"

### SUMMARY

Poly-lactic acid (PLA) resins are derived from 100% plants, such as starch and sugar as raw material to be environment-friendly synthetic resins not at all derived from petroleum based raw material. The inventors use starch, sugar, etc. contained in some types of food waste such as shochu lees (e.g. rice, sweet potato, wheat, and buckwheat shochu lees), okara (e.g. tofu refuse), rice bran, wheat bran, corn, potato, sweet potato, or sugarcane to manufacture biodegradable green plastic products.

PLA green plastics should not be biodegraded in a special soil environment, preferably in a general soil environment for a short period. Moreover, the raw material of biodegradable green plastic products is desirably organic waste material (e.g., shochu lees) discharged in large amounts from factories, etc., without limitation to shochu lees, sake lees, corn, sugarcane, potato, bamboo, etc. On the other hand, for example, the cost of the machines for methane fermentation of shochu lees tends to increase. Such machines are too expensive to be introduced in a small-scale shochu plant.

Moreover, conventional biodegradable plastics are partially (15-25% of the raw material) decarbonize as biodegradable biomass but incompletely biodegradable because the petroleum part accounts for more than a half of them.

The present invention has been created in view of the above-mentioned previously existing circumstances. The objective of the present invention is to provide a method and a system of manufacturing a biodegradable plastic product that is made of an organic substance as raw material and biodegraded in a general soil environment for a short period.

The first aspect of the present invention provides a method of manufacturing a biodegradable plastic product, including:
a first step of crushing organic waste material;
a second step of adding an inorganic treatment agent and a dehydrating treatment agent to organic waste material with a high water content that is hardly dehydrated but required to be crushed and stirring the mixture to dehydrate and solidify the organic waste material;
a third step of drying the solidified organic waste material and powdering the dried organic waste material to microparticles;
a fourth step of subjecting the powdered organic waste material to lactic acid generation; and
a fifth step of molding a biodegradable plastic product with molding equipment by using the poly-lactic acid organic waste material as raw material.

The second aspect of the present invention provides the method of manufacturing a biodegradable plastic product according to the first aspect of the present invention, in which, in the second step, the mixture is stirred with a predetermined auxiliary material.

The third aspect of the present invention provides the method of manufacturing a biodegradable plastic product according to the first or the second aspect of the present invention, in which the inorganic treatment agent is powder containing silicon.

The fourth aspect of the present invention provides the method of manufacturing a biodegradable plastic product according to any one of the first to the third aspect of the present invention, further including the sixth step of mixing cellulose nanofiber with the poly-lactic acid organic waste material, in which, in the fifth step, the biodegradable plastic product is molded by using the mixture of the poly-lactic acid organic waste material with the cellulose nanofiber as raw material.

The fifth aspect of the present invention provides the method of manufacturing a biodegradable plastic product according to any one of the first to the fourth aspect of the present invention, further including the seventh step of separating organic waste material before the first step and crushing each one of the separated organic waste material and the eighth step of mixing each one of the solidified organic waste material together after the second step, in which, in the third step, the solidified organic waste material mixed in the eighth step is dried and powdered to microparticles.

The sixth aspect of the present invention provides the method of manufacturing a biodegradable plastic product according to any one of the first to the fifth aspect of the present invention, in which, the fourth step includes adding cellulose to the poly-lactic acid organic waste material.

The seventh aspect of the present invention provides the method of manufacturing a biodegradable plastic product according to any one of the first to the sixth aspect of the present invention, in which, the biodegradable plastic product is a packaging plastics product, a plastic bottle, a food tray, a film product, or a multi-film.

The eighth aspect of the present invention provides a system of manufacturing a biodegradable plastic product, including:
a first device of crushing organic waste material;
a second device of adding an inorganic treatment agent and a dehydrating treatment agent to the crushed organic waste material and stirring the mixture to dehydrate and solidify the organic waste material;
a third device of drying the solidified organic waste material and powdering the dried organic waste material to microparticles;
a fourth device of subjecting the powdered organic waste material to lactic acid generation; and
a fifth device of molding a biodegradable plastic product with molding equipment by using the poly-lactic acid organic waste material as raw material.

According to the present invention, the method and the system crushes organic waste material, adds an inorganic treatment agent and a dehydrating treatment agent to the crushed organic waste material and stirs the mixture to dehydrate and solidify the organic waste material, dries the solidified organic waste material and powders the dried organic waste material to microparticles, subjects the powdered organic waste material to poly-lactic acid generation, and molds a biodegradable plastic product with molding equipment by using the poly-lactic acid organic waste material as raw material.

Accordingly, a biodegradable plastic product to be biodegraded in a general soil environment for a short period can be manufactured by using any organic substances as raw material.

### BRIEF DESCRIPTION OF THE DRAWINGS

FIG. 1 is a flow chart that shows an example manufacturing flow of a biodegradable plastic product according to one embodiment of the present invention.
FIG. 2 is a conceptual diagram that shows an overall configuration of the system of manufacturing a biodegradable plastic product according to the embodiment.
FIG. 3 shows an example packaging container manufactured through the embodiment.
FIG. 4 shows an example plastic bag manufactured through the embodiment.
FIG. 5 shows an example of microparticles powdered in the powdering process according to the embodiment.

### DETAILED DESCRIPTION

The present invention is a manufacturing technology of a soil reducing poly-lactic acid (PLA) biodegradable plastic product by using various organic waste materials, for example, 100% natural resources such as shochu lees such as rice, wheat, sweet potato, or buckwheat lees, refined sake lees, tofu refuse, rice bran, draff, sugarcane dreg, corn, potato, and various grains. The poly-lactic acid is a synthetic plastic derived from a plant to produce lactic acid by fermentation in which lactic acid bacteria act on glucose or sugar (sucrose) contained in the starch of raw material.

The present invention is also a manufacturing technology of a biodegradable plastic product molded by mixing cellulose nanofiber (CNF) removed from the organic waste material of fibered material obtained from trees, bamboo, paper, etc. Poly-lactic acid (PLA) biodegradable plastic material and cellulose nanofiber (CNF) are prorated so that biodegradable plastics with different strengths and thicknesses can be produced. Accordingly, various products can be manufactured by changing the proration depending on the product (use).

For example, the biodegradable plastic product manufactured by the technology of the present invention has water-soluble, which dissolves at a normal temperature and a high temperature of 65 °C or more. Finally, the biodegradable plastic product is decomposed into water and carbon dioxide by microorganisms in agricultural soil, etc.

### Basic manufacturing process

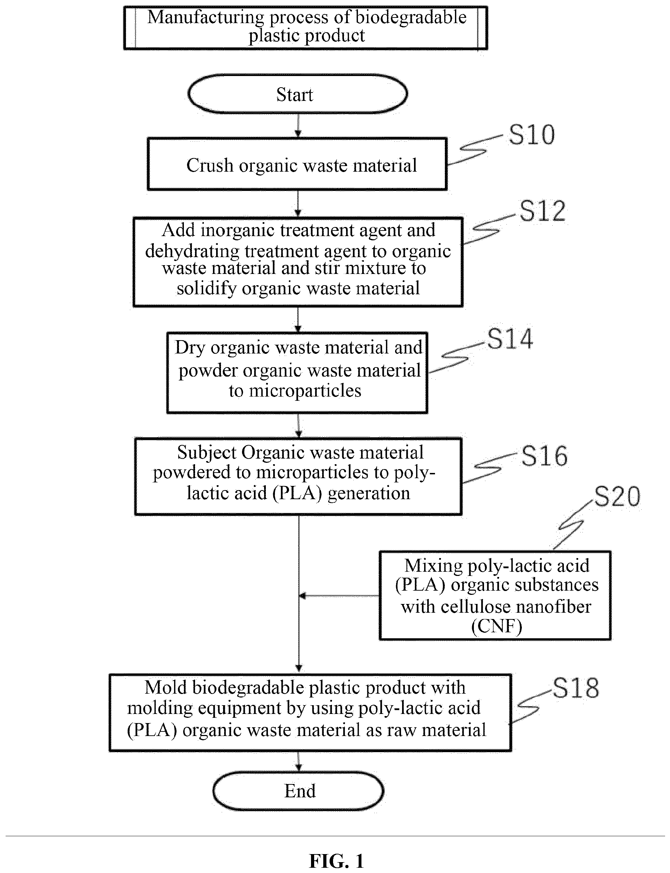

FIG. 1 is a flow chart that shows the flow chart of a basic manufacturing method of a biodegradable plastic product according to one embodiment of the present invention. First, organic waste material is crushed (Step S10). The organic waste materials, for example, 100% natural resources such as shochu lees such as rice, wheat, sweet potato, or buckwheat lees, refined sake lees, tofu refuse, rice bran, draff, sugarcane dreg, corn, potato, and various grains.

An inorganic treatment agent and a dehydrating treatment agent are added to the crushed organic waste material and the mixture is stirred to dehydrate and solidify the organic waste material (Step S12). The dehydration and solidification are carried out on shochu lees and draff with a high water content. After detoxified by adding an inorganic treatment agent, the organic waste material is mixed with auxiliary material as required. In Step S14, the solidified organic waste material is dried and powdered to microparticles. For example, the size of the organic waste material becomes about 40 microns or more if the organic waste material is crushed with a crusher.

The inorganic treatment agent is powder containing mineral such as silicon (e.g. coral). The inorganic treatment agent is added to an organic waste material with a high water content, and the mixture is stirred to dehydrate the organic waste material. The organic waste material is then dried and solidified. The silicon is suitable as fertilizer, which turns to soil nutrition after the manufactured biodegradable plastic product is buried in the soil. For example, shochu contains water of 93% and nutrient of 7%. It is important to absorb water from shochu lees. The inorganic treatment agent of the embodiment absorbs water from organic waste material to make it possible to dry and solidify the organic waste material. At this time, auxiliary material is added to the original raw material (organic waste material) when the starch is insufficient. For example, the auxiliary material is rice bran, tofu refuse, etc. The auxiliary material is adjusted and added because the amount of the starch varies by raw material.

The organic waste material powdered to microparticles in Step S14 is subjected to poly-lactic acid (PLA) generation (Step S16). The poly-lactic acid is a synthetic plastic derived from a plant to produce lactic acid by fermentation in which lactic acid bacteria act on glucose or sugar (sucrose) contained in the starch of raw material.

In Step S18, a biodegradable plastic product is molded with molding equipment by using the poly-lactic acid (PLA) organic waste material as raw material. At this time, Step S20 of mixing the poly-lactic acid (PLA) biodegradable plastic material with cellulose nanofiber (CNF) may be added. Then, a biodegradable plastic product with different strengths and thicknesses is molded with molding equipment by using the mixture as raw material. Therefore, various products can be manufactured by changing the proration depending on the product (use).

### Configuration of system of manufacturing biodegradable plastic product

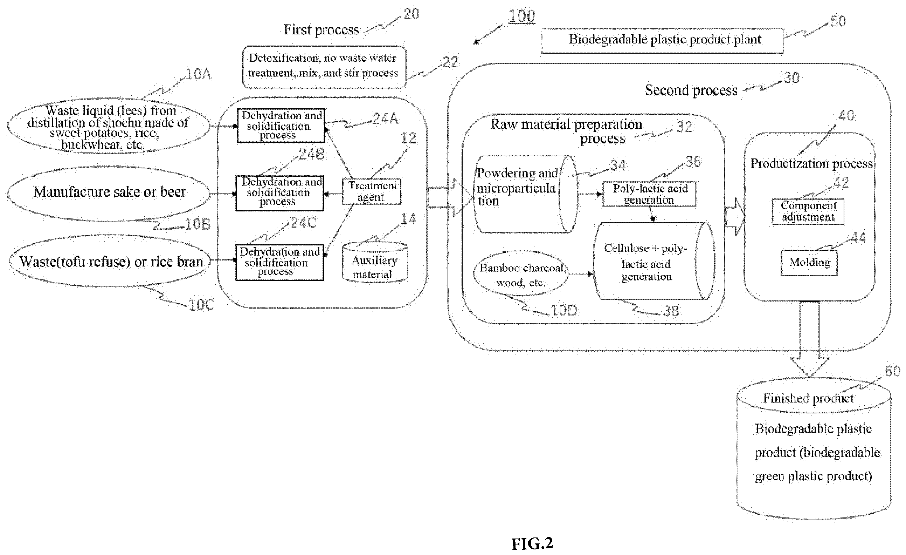

The overall configuration of the system of manufacturing a biodegradable plastic product according to the embodiment is explained below with reference to FIG. 2. FIG. 2 is the overall schematic diagram of a manufacturing system. The manufacturing system 100 according to the embodiment includes a first process 20 of subjecting organic waste material to detoxification, no waste water treatment, mix, and stir and a second process 30 including a raw material preparation process 32 and a productization process 40.

Before the first process 20, the two or more types of organic waste material are separated to subject each one of the separated organic waste material to the first process. For example, the organic waste material 10A is waste liquid (lees) from distillation of shochu made of sweet potato, rice, buckwheat, etc., which is dehydrated and solidified by adding the inorganic treatment agent 12 and stirring the mixture (dehydration and solidification process 24A). The organic waste material 10B is lees or draff generated when sake or beer is manufactured, which is dehydrated and solidified by adding the inorganic treatment agent 12 and stirring the mixture (dehydration and solidification process 24B). The organic waste material 10C is the other organic waste material (e.g. tofu refuse, rice bran), which is dehydrated and solidified by adding the inorganic treatment agent 12 and stirring the mixture (dehydration and solidification process 24C).

In the above-mentioned dehydration and solidification processes 24A-24C, auxiliary material 14 such as rice bran or tofu refuse is added to the original raw material (organic waste material) when the starch is insufficient. The auxiliary material 14 is adjusted and added because the amount of the starch varies by raw material.

The organic waste material dehydrated and solidified as described above is, for example, delivered to a biodegradable plastic product plant 50 and subjected to the second process 30. In the raw material preparation process 32, the organic waste materials 10A-10C are dried and powdered to microparticles (powdering and microparticulation process 34). The microparticulate organic waste material is subjected to poly-lactic acid (PLA) generation (poly-lactic acid generation process 36).

If necessary, in the raw material preparation process 32, cellulose nanofiber (CNF) is removed from fibered organic waste material 10D such as bamboo charcoal or wood and mixed with the poly-lactic acid (PLA) organic waste material (cellulose + poly-lactic acid organic waste material mixing process 38). Various biodegradable plastic products with different strengths and thicknesses can be produced by changing the composition amount of the cellulose nanofiber and the poly-lactic acid (PLA) organic waste material.

The poly-lactic acid organic waste material is subjected to the productization process 40 to adjust the component (component adjustment process 42), and molded with a predetermined molding machine (molding process 44) to obtain a biodegradable plastic product as a finished product 60. For the molding, various known molding techniques (e.g., T-die method, vacuum and pressure forming, press molding, blow molding, and injection molding) that is appropriate for products to be molded can be adopted.

The patterns of the biodegradable plastic product as a finished product 60 includes for example, various biodegradable plastic products (e.g. plastic bottle, various biodegradable plastic bags, and packaging plastics product) as the pattern A, products for food manufacturing, processing business, supermarkets, and department store (e.g. food packaging tray, disposable lunch box) as the pattern B, and various biodegradable plastics for agriculture (e.g. multi-film, pots, biodegradable plastic for various greenhouses) as the pattern C.

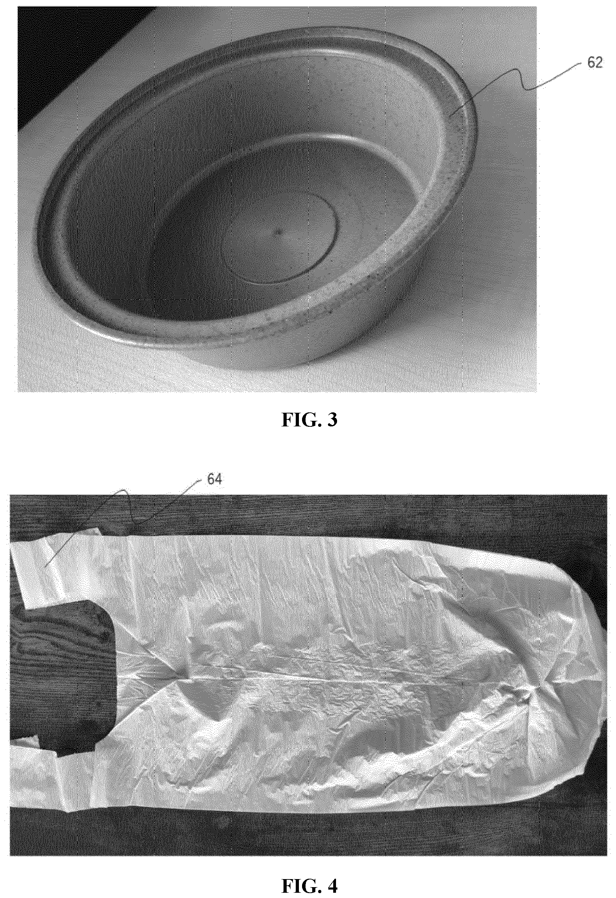

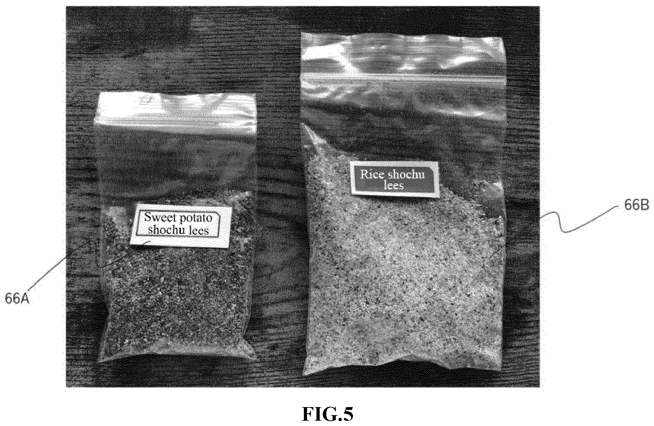

FIG. 3 shows an example packaging container 62 manufactured by the manufacturing system 100 according to the embodiment. FIG. 4 shows an example plastic bag 64 manufactured by the manufacturing system 100 according to the embodiment. FIG. 5 shows an example of microparticles powdered in the powdering process according to the embodiment. The microparticles 66A and the microparticles 66B are produced from sweet potato shochu lees and rice shochu lees, respectively, as raw material.

### Effect

According to the embodiment described above, organic waste material is crushed, and an inorganic treatment agent and a dehydrating treatment agent are added to the crushed organic waste material, and the mixture is stirred to dehydrate and solidify the organic waste material. The solidified organic waste material is dried and powdered to microparticles, and the powdered organic waste material is subjected to lactic acid generation. A biodegradable plastic product is molded with molding equipment by using the poly-lactic acid organic waste material as raw material. Accordingly, a biodegradable plastic product to be biodegraded in a general soil environment for a short period can be manufactured by using any organic substances as raw material.

The embodiment described above is one example, which can be appropriately modified within producing a similar effect. The organic waste material shown in the above-mentioned embodiment is one example. Any types of plant-derived organic waste, for example, seaweed, food waste, rice lees from refined sake, and sugar cane can be used as raw material. In the above-mentioned embodiment, the poly-lactate acid organic waste material is mixed with cellulose nanofiber and molded, but this is also one example, The cellulose nanofiber only has to be mixed as required, and the mixture fraction may be appropriately change as usage. The molding technique in the embodiment is also one example. Various known plastic molding techniques are applicable. The product described above is also one example. Various biodegradable plastic products can be manufactured.

### Industrial applicability

According to the present invention, organic waste material is crushed, and an inorganic treatment agent and a dehydrating treatment agent are added to the crushed organic waste material, and the mixture is stirred to dehydrate and solidify the organic waste material. The solidified organic waste material is dried and powdered to microparticles, and the powdered organic waste material is subjected to lactic acid generation. A biodegradable plastic product is molded with molding equipment by using the poly-lactic acid organic waste material as raw material.

Accordingly, the present invention is suitable as a method and a system of manufacturing a biodegradable plastic product to be biodegraded in a general soil environment for a short period by using any organic substances as raw material.

### Reference Signs List

10A-10D: Organic waste material
12: Treatment agent
14: Auxiliary material
20: First process
22: Detoxification, no waste water treatment, mix, and stir process
24A-24C: Dehydration and solidification process
30: Second process
32: Raw material preparation process
34: Powdering and microparticulation process
36: Poly-lactic acid generation process
38: Process of mixing poly-lactic acid organic waste material with cellulose nanofiber
40: Productization process
42: Component adjustment process
44: Molding process
50: Biodegradable plastic product plant
60: Finished product
62: Packaging container
64: Plastic bag
66A, 66B: Microparticles
100: Manufacturing system

## Claims

1. A method of manufacturing a biodegradable plastic product, comprising:
a first step of crushing organic waste material;
a second step of adding an inorganic treatment agent and a dehydrating treatment agent to the crushed organic waste material and stirring the mixture to dehydrate and solidify the organic waste material;
a third step of drying the solidified organic waste material and powdering the dried organic waste material to microparticles;
a fourth step of subjecting the powdered organic waste material to lactic acid generation; and
a fifth step of molding a biodegradable plastic product with molding equipment by using the poly-lactic acid organic waste material as raw material.

2. The method of manufacturing a biodegradable plastic product according to claim 1, wherein, in the second step, the mixture is stirred with a predetermined auxiliary material.

3. The method of manufacturing a biodegradable plastic product according to claim 1 or 2, wherein, the inorganic treatment agent is powder containing silicon.

4. The method of manufacturing a biodegradable plastic product according to any one of claims 1 to 3, further comprising:
the sixth step of mixing cellulose nanofiber with the poly-lactic acid organic waste material, wherein,
in the fifth step, the biodegradable plastic product is molded by using the mixture of the poly-lactic acid organic waste material with the cellulose nanofiber as raw material.

5. The method of manufacturing a biodegradable plastic product according to any one of claims 1 to 4, further comprising:
the seventh step of separating organic waste material before the first step and crushing each one of the separated organic waste material; and
the eighth step of mixing each one of the solidified organic waste material together after the second step, wherein,
in the third step, the solidified organic waste material mixed in the eighth step is dried and powdered to microparticles.

6. The method of manufacturing a biodegradable plastic product according to any one of claims 1 to 5, wherein, the fourth step includes adding cellulose to the poly-lactic acid organic waste material.

7. The method of manufacturing a biodegradable plastic product according to any one of claims 1 to 6, wherein, the biodegradable plastic product is a packaging plastics product, a plastic bottle, a food tray, a film product, or a multi-film.

8. A system of manufacturing a biodegradable plastic product, comprising:
a first device of crushing organic waste material;
a second device of adding an inorganic treatment agent and a dehydrating treatment agent to the crushed organic waste material and stirring the mixture to dehydrate and solidify the organic waste material;
a third device of drying the solidified organic waste material and powdering the dried organic waste material to microparticles;
a fourth device of subjecting the powdered organic waste material to lactic acid generation; and
a fifth device of molding a biodegradable plastic product with molding equipment by using the poly-lactic acid organic waste material as raw material.
